# EUROPÄISCHE PATENTSCHRIFT

(11) **EP 1 617 212 B1**
(45) Veröffentlichungstag und Bekanntmachung des Hinweises auf die Patenterteilung: **22.08.2007**
(21) Anmeldenummer: 04016378.4
(22) Anmeldetag: 13.07.2004
(51) Int. Cl.: G01N 27/22, G01N 33/28, F04D 13/08

(54) **Kapazitiver Sensor zum Erfassen von Wasser in Öl**
Capacitive sensor for detecting water in oil
Capteur capacitif pour détecter de l'eau dans l'huile

(43) Veröffentlichungstag der Anmeldung: 18.01.2006
(73) Patentinhaber: GRUNDFOS A/S, DK-8850 Bjerringbro (DK)
(72) Erfinder: Nybo, Peter Jungklas, 8900 Randers (DK); Yli-Korpela, Heikki, 01680 Vantaa (FI); Ilves, Lasse, 00940 Helsinki (FI); Christensen, Lars, 8870 Langaa (DK); Nielsen, Knud Georg, 8800 Viborg (DK)
(74) Vertreter: Hemmer, Arnd

(56) Entgegenhaltungen:
- EP-A- 0 701 110
- DE-A- 1 653 752
- DE-A- 10 155 371
- GB-A- 675 157
- US-A- 2003 020 494
- US-A1- 2003 141 882

## Beschreibung

Es ist bekannt, kapazitive Sensoren zum Erfassen eines Fremdstoffes in einem Fluid einzusetzen. Derartige Sensoren werden beispielsweise eingesetzt, um Wasser in Öl detektieren zu können. Dies ist unter anderem bei in Wasser eintauchenden Motoren, wie sie bei Tauchpumpen verwendet werden, wichtig. Diese Motoren weisen zur Abdichtung eine Ölkammer bzw. Ölvorlage auf, welche verhindert, dass Wasser direkt in das Innere des Motors eindringen kann. Wichtig ist jedoch, frühzeitig zu erfassen, ob durch Verschleiß einer Dichtung Wasser in die Ölkammer eintritt, um den Motor bzw. die Pumpe rechtzeitig außer Betrieb nehmen und reparieren zu können, bevor das Wasser auch in das Innere des Motors eindringt.

US 2003/0141882 A1 offenbart einen Sensor zur Überwachung eines Fluidzustandes, welcher beispielsweise im Bereich der Schmierung einer Maschine oder einer Hydraulikanlage eines Kraftfahrzeuges zum Einsatz kommen kann. Dieser Sensor weist eine gelochte äußere Elektrode und eine stabförmige und im Inneren angeordnete zylindrische Elektrode auf, wobei der Freiraum zwischen beiden Elektroden von dem zu überwachenden Fluid durchströmt wird. Ferner ist dieser Sensor noch von einer äußeren Abschirmung umgeben, welche ebenfalls mit Eintrittsöffnung für das Fluid versehen ist. Zu einem axialen Ende hin sind die Elektroden und die Abschirmung stiftförmig verlängert, wobei diese Stifte dann zum elektrischen Anschluss dienen.

GB 675,157 offenbart eine Vorrichtung zur Überwachung der Veränderungen der elektrischen Leitfähigkeit von Flüssigkeiten, welche kapazitiv erfolgen soll. Hierzu ist ein Sensor mit zwei Elektroden vorgesehen, wobei die äußere Elektrode zylindrisch und die innere Elektrode stiftförmig im Zentrum der äußeren Elektrode ausgebildet ist. Beide Elektroden sind an einer scheibenförmigen Platte aus Isoliermaterial angebracht, welche sich an einer Stirnseite der Elektroden erstreckt.

US 2003/0020494 A1 offenbart einen kapazitiven Kraftstoffsensor, welcher zwei rohrförmige ineinanderliegend angeordnete Elektroden aufweist. Die äußere Elektrode ist mit Öffnungen versehen, durch welche der Kraftstoff in einen ringförmigen Freiraum zwischen der äußeren und der inneren Elektrode eintreten kann. Im Inneren der inneren Elektrode ist eine Leiterplatte mit elektronischen Bauteilen angeordnet. Die Leiterplatte ist elektrisch sowohl mit der inneren als auch mit der äußeren Elektrode verbunden. Dazu ist die Stirnseite der inneren Elektrode offen ausgebildet und es erstrecken sich durch diese Öffnung Anschlusskontakte zu der geschlossenen Stirnseite der äußeren Elektrode. Um den ringförmigen Freiraum zwischen den beiden Elektroden gegenüber der Öffnung an der Stirnseite der inneren Elektrode abzudichten, sind nahe dem stirnseitigen Ende der inneren Elektrode O-Ringe zwischen der äußeren und der inneren Elektrode angeordnet.

Es ist Aufgabe der Erfindung einen verbesserten Sensor zum Erfassen eines Fremdstoffes in einem Fluid zu schaffen, welcher einen vereinfachten Aufbau und einen vereinfachten elektrischen Anschluss ermöglicht und einen verbesserten Fluidaustausch im Messbereich des Sensors zulässt.

Diese Aufgabe wird durch einen kapazitiven Sensor durch die in Anspruch 1 angegebenen Merkmale gelöst. Bevorzugte Ausführungsformen ergeben sich aus den Unteransprüchen.

Der erfindungsgemäße kapazitive Sensor ist zum Erfassen eines Fremdstoffes in einem Fluid, beispielsweise von Wasser in Öl, vorgesehen. Der Sensor weist eine rohrförmige erste Elektrode sowie eine zweite Elektrode auf, welche im Inneren der ersten Elektrode derart angeordnet ist, dass die Außenfläche der zweiten Elektrode von der Innenwandung der rohrförmigen ersten Elektrode beabstandet ist. Auf diese Weise wird im Inneren der ersten Elektrode vorzugsweise ein ringförmiger Freiraum zwischen der Innenwandung der rohrförmigen Elektrode und der zweiten im Inneren angeordneten Elektrode gebildet. In diesem Freiraum wird ein elektrisches Feld zwischen den beiden Elektroden erzeugt. Ferner ist der Freiraum so ausgebildet, dass das durch den Sensor zu über wachende Fluid in diesen eindringt. Das Fluid bildet somit das Dielektrikum zwischen den beiden Elektroden. Wenn sich der Zustand des Fluids, beispielsweise durch Eindringen eines Fremdstoffes ändert, verändern sich dessen dielektrischen Eigenschaften, so dass das elektrische Feld zwischen den Elektroden verändert wird, was in bekannter Weise messtechnisch erfasst werden kann.

Die rohrförmige Elektrode weist vorzugsweise einen kreisförmigen Querschnitt auf, in dessen Zentrum die zweite Elektrode derart angeordnet ist, dass die Oberfläche der zweiten Elektrode im Wesentlichen in allen radialen Richtungen gleich weit vom Innenumfang der rohrförmigen Elektrode beabstandet ist. Vorzugsweise ist die zweite Elektrode stabförmig ausgebildet und erstreckt sich zentral im Inneren der rohrförmigen Elektrode in deren Längsrichtung.

Die zweite Elektrode ist an einer Leiterplatte zum elektrischen Anschluss angebracht, welche im Inneren der ersten rohrförmigen Elektrode angeordnet ist. Dabei ist die Leiterplatte vorzugsweise so angeordnet, dass sie sich in einer von der Längsachse sowie der Durchmesserachse der rohrförmigen Elektrode aufgespannten Ebene erstreckt. Dazu weist die Leiterplatte vorzugsweise eine Breite auf, welche dem Innendurchmesser der äußeren Elektrode entspricht, so dass die Leiterplatte formschlüssig im Inneren der äußeren Elektrode fixiert werden kann. Die zweite Elektrode ist fest an der Leiterplatte 14 angebracht und erstreckt sich ausgehend von dieser entlang der Mittel- bzw. Längsachse der rohrförmigen ersten Elektrode. Auf diese Weise sorgt die Leiterplatte nicht nur für den elektrischen Anschluss, sondern auch für die mechanische Fixierung der zweiten Elektrode im Inneren der ersten rohrförmigen Elektrode. Auf der Leiterplatte können darüber hinaus zusätzliche elektronische Bauteile angeordnet werden.

Die rohrförmige erste Elektrode ist an einer Stirnseite offen ausgebildet. Durch die offene Stirnseite kann das Fluid, in welches der Sensor eingetaucht ist, und in welchem die Fremdstoffe detektiert werden sollen, in das Innere der ersten rohrförmigen Elektrode eintreten.

Die zweite Elektrode ist dabei im Inneren der ersten Elektrode so angeordnet, dass sie sich zu der offenen Stirnseite hin erstreckt. Das bedeutet, die zweite Elektrode weist ein freies Stirnende auf, welches der offenen Stirnseite der ersten Elektrode zugewandt ist oder im Bereich der stirnseitigen Öffnung der ersten Elektrode angeordnet ist. So ist ein ringförmiger Freiraum an der Stirnseite des Sensors gebildet, durch den das Fluid ein- und austreten kann.

Vorzugsweise ist die erste und/oder die zweite Elektrode von einer Isolierung umhüllt. Diese Isolierung ist vorzugsweise als elektrische Isolierung aus einem elektrisch nicht leitenden Material ausgebildet, um einen Kurzschluss zwischen den beiden Elektroden, beispielsweise bei Kontakt mit Wasser, zu verhindern. Darüber hinaus kann die Isolierung als Korrosionsschutz für die Elektroden dienen.

Weiter bevorzugt ist die zweite Elektrode von einer Isolierung in Form eines, vorzugsweise an einem Ende geschlossenen, Glasrohres umhüllt. Dabei ist die zweite, innere Elektrode im Inneren des Glasrohres angeordnet, welches wiederum im Inneren der rohrförmigen ersten Elektrode so angeordnet ist, dass es von dessen Innenwandungen beabstandet ist. So kann durch die erste und die zweite Elektrode in dem Freiraum zwischen dem Außenumfang des Glasrohres und dem innenumfang der ersten Elektrode ein elektrisches Feld erzeugt werden. Das Glas als Isoliermaterial bietet zum einen eine gute elektrische Isolierung, zum anderen einen guten Korrosionsschutz und das elektrische Feld wird wenig gestört.

Alternativ oder zusätzlich kann in der rohrförmigen ersten Elektrode zumindest eine umfängliche Öffnung vorgesehen sein, durch welche das Fluid in das Innere der Elektrode bzw. des Sensors eintreten kann. Wenn in der ersten Elektrode eine umfängliche und eine stirnseitige Öffnung vorgesehen sind, kann das Fluid die innere Elektrode durch beide Öffnungen frei durchströmen, so dass ein guter Fluidaustausch im Inneren des Sensors erreicht wird, um Fremdstoffe möglichst frühzeitig detektieren zu können. Besonders bevorzugt sind am Umfang der ersten Elektrode mehrere Öffnungen ausgebildet und vorzugsweise gleichmäßig über den Umfang verteilt, so dass ein gleichmäßiger und stetiger Austausch des Fluids in allen Bereichen des Sensors im Inneren der ersten Elektrode erreicht werden kann.

Bevorzugt ist ein Bereich im Inneren der ersten Elektrode, in welchem elektrische Anschlüsse und insbesondere eine Leiterplatte angeordnet sind, mit einem Isolationsmaterial ausgegossen. Dabei ist die zweite Elektrode bevorzugt zumindest teilweise außerhalb des vergossenen Bereiches angeordnet. Durch das Vergießen mit Isolationsmaterial, beispielsweise einem Kunstharz wie Epoxydharz, kann zum einen die zweite Elektrode mit der Leiterplatte sicher im Inneren der ersten rohrförmigen Elektrode fixiert werden. Zum anderen werden die elektrischen Bauteile und elektrischen Anschlüsse an der Leiterplatte und der ersten Elektrode gleichzeitig durch das Vergussmaterial elektrisch isoliert. In dem Bereich, in dem die zweite Elektrode außerhalb des vergossenen Bereiches angeordnet ist, liegt der eigentliche Messbereich des Sensors, in dem das Fluid zwischen der ersten und zweiten Elektrode eindringen kann. D. h. in diesem Bereich besteht ein Freiraum zwischen beiden Elektroden, während der Raum zwischen den beiden Elektroden in dem vergossenen Bereich mit Vergussmasse gefüllt ist.

Vorzugsweise ist in den vergossenen Bereich die die zweite Elektrode umhüllende Isolierung und/oder zumindest ein Zuleitungskabel eingegossen. Die Isolierung der zweiten Elektrode greift somit dichtend in die Vergussmasse ein. Auf diese Weise kann auf zusätzliche Dichtelemente verzichtet werden. Besonders bevorzugt ist, wie oben beschrieben, die Isolierung der zweiten Elektrode als einseitig geschlossenes Glasrohr ausgebildet. Bei dieser Ausführungsform greift das offene Ende des Glasrohres in die Vergussmasse ein, so dass dieser Bereich durch die Vergussmasse geschlossen und nach außen abgedichtet wird. Zusätzlich oder alternativ können auch Zuleitungskabel zu der ersten und/oder zweiten Elektrode bzw. der Leiterplatte mit eingegossen werden. Damit werden die Zuleitungskabel zum einen im Inneren des Sensors bzw. der ersten Elektrode fixiert und zum anderen eine nach außen abgedichtete Verbindung der Zuleitungskabel mit dem Sensor geschaffen.

Weiter bevorzugt weist ein stirnseitiger Endbereich der ersten Elektrode eine Öffnung auf, durch welche sich zumindest eine elektrische Anschlussleitung bzw. zumindest ein elektrisches Zuleitungskabel in das Innere der ersten Elektrode erstreckt. Diese Öffnung ist dabei vorzugsweise an dem der stirnseitigen Eintrittsöffnung für das Fluid abgewandten Seite des Sensors vorgesehen. Die elektrische Anschlussleitung kann im Bereich der Öffnung durch eine zusätzliche Dichtung oder durch die im Inneren der Elektrode vorgesehene Vergussmasse abgedichtet und gegebenenfalls zusätzlich durch eine Klemmverbindung gesichert sein.

Zweckmäßigerweise ist die erste Elektrode durch eine Klemmverbindung elektrisch leitend mit einer elektrischen Anschlussleitung verbunden. So kann die elektrische Anschlussleitung beispielsweise so ausgebildet sein, dass sie einen äußeren Leiter beispielsweise in Form eines ringförmigen Drahtgeflechtes aufweist, welcher durch Verklemmen mit der rohrförmigen ersten Elektrode verbunden wird.

Besonders bevorzugt ist der Sensor zum Einbau mit der rohrförmigen Elektrode in eine korrespondierende Öffnung eines Gehäuses dichtend einsetzbar. Dazu kann die rohrförmige Elektrode an ihrem Außenumfang entsprechende Eingriffs und/oder Dichtmittel aufweisen. Beispielsweise kann am Außenumfang der Elektrode ein entsprechender Absatz, radial nach außen vorstehender Flansch und/oder ein Gewinde ausgebildet sein, um in eine korrespondierende Ausnehmung bzw. Gewindebohrung in dem Gehäuse eingesetzt bzw. eingeschraubt zu werden. Zusätzlich kann eine Dichtung, beispielsweise ein O-Ring vorgesehen sein, um die rohrförmige Elektrode gegenüber dem Gehäuse abzudichten. Dabei wird die rohrförmige Elektrode vorzugsweise so in eine Wandung eines Gehäuses eingesetzt, dass die Eintrittsöffnungen für das Fluid im Inneren des Gehäuses angeordnet sind und die Öffnung, durch die sich die elektrischen Anschlussleitungen nach außen erstrecken, außerhalb des Gehäuses angeordnet sind. Dabei ist die rohrförmige Elektrode besonders bevorzugt von außen in das Gehäuse einsetzbar, so dass sie leicht ausgetauscht werden kann. Ferner ist es möglich, die Elektrode in eine ohnehin vorhandene Gehäuseöffnung, beispielsweise zum Einfüllen eines Fluids, wie beispielsweise Öl, einzusetzen.

Besonders bevorzugt wird der kapazitive Sensor gemäß der vorangehenden Beschreibung in einer Flüssigkeitsvorlage eines in ein Fluid eintauchbaren Elektromotors verwendet. Ein solcher Elektromotor wird beispielsweise in einer Tauchpumpe eingesetzt. Dabei ist in dem Bereich des Elektromotors, in dem die Welle aus dem Statorgehäuse austritt, eine Flüssigkeitsvorlage in einer Kammer vorgesehen, welche das Innere des Elektromotors von dem umgebenden Fluid trennt. Diese Flüssigkeitsvorlage weist zwei Dichtungen auf, eine zum trockenen Inneren des Elektromotors hin und eine zum umgebenden Fluid hin. Wenn durch Verschleiß der äußeren Dichtung das umgebende Fluid in die Flüssigkeitsvorlage eintritt, kann dies durch den erfindungsgemäßen Sensor erfasst werden, bevor auch die innere Dichtung verschleißt und das Fluid in das Innere des Motors eintritt. Die Flüssigkeitsvorlage weist bevorzugt Öl auf und das umgebende Fluid kann beispielsweise Wasser sein. Durch den erfindungsgemäßen Sensor kann dann ein Wassereintritt in die Flüssigkeits- bzw. Ölvorlage frühzeitig erfasst werden, bevor die Gefahr besteht, dass das Wasser auch in das Innere des Elektromotors eindringt und diesen zerstört. Eine elektrisch angetriebene Tauchpumpe wird z.B. in DE 1653752 beschrieben.

Nachfolgend wird die Erfindung beispielhaft anhand der beigefügten Zeichnungen beschrieben. In diesen zeigen:
- Fig. 1: eine Explosionsansicht eines kapazitiven Sensors gemäß der Erfindung,
- Fig. 2: eine Seitenansicht des zusammengesetzten Sensors gemäß Fig. 1 und
- Fig. 3: einen Längsschnitt durch den zusammengesetzten Sensor gemäß Fig. 2.

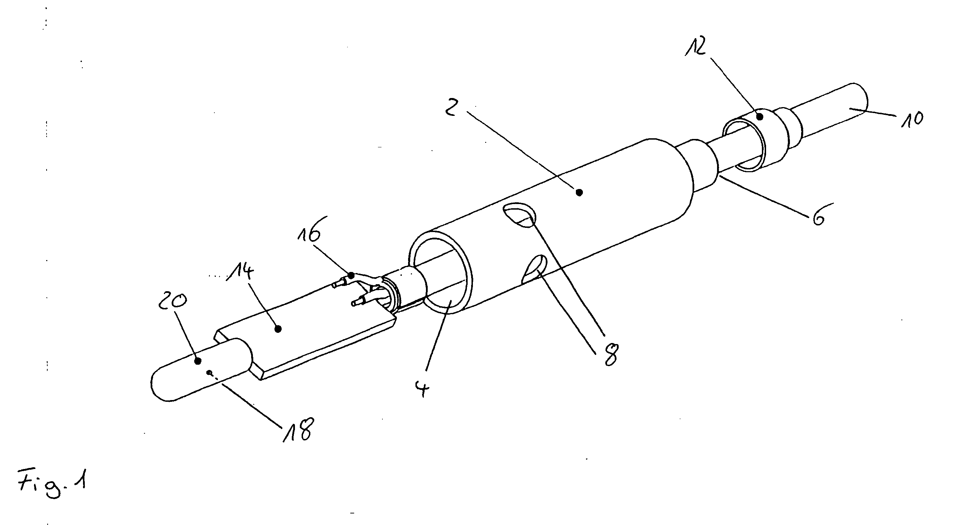

Der in Fig. 1 gezeigte Sensor weist eine rohrförmige äußere Elektrode 2 mit kreisförmigem Querschnitt auf. Die erste äußere Elektrode 2 weist zwei stirnseitige Öffnungen 4 und 6 sowie umfängliche Öffnungen 8 auf. Durch die stirnseitige Öffnung 6 erstreckt sich eine elektrische Anschlussleitung 10 in das Innere der äußeren Elektrode 2. Die elektrische Anschlussleitung 10 wird im Bereich der stirnseitigen Öffnung 6 durch Verklemmen mit der äußeren Elektrode 2 mechanisch verbunden. Dabei erfolgt gleichzeitig eine elektrische Kontaktierung mit einem äußeren Leiter in der elektrischen Anschlussleitung 10. Die Klemmverbindung im Bereich der stirnseitigen Öffnung 6 wird durch einen Isolationsschlauch 12, beispielsweise einen Schrumpfschlauch, nach außen isoliert.

Im Inneren der äußeren Elektrode 2 ist eine Leiterplatte 14 angeordnet, welche mit weiteren elektrischen Leitern 16 im Inneren der elektrischen Anschlussleitung 10 verbunden ist. Die Leiterplatte 14 erstreckt sich in einer von der Längsachse der äußeren Elektrode 2 sowie deren Durchmesserachse aufgespannten Ebene. An der der stirnseitigen Öffnung 4 der äußeren Elektrode 2 zugewandten Stirnkante der Leiterplatte 14 ist eine zweite Elektrode 18 (in Fig. 1 nicht gezeigt) angebracht. Die zweite innere Elektrode 18 erstreckt sich ausgehend von der Stirnkante der Leiterplatte 14 in Längsrichtung der äußeren Elektrode 2 zu der stirnseitigen Öffnung 4 der äußeren Elektrode 2 hin. Dabei ist die zweite Elektrode 18 mittig im Inneren der äußeren Elektrode 2 angeordnet, so dass sie von der inneren Wandung der äußeren Elektrode 2 über ihren ganzen Umfang radial gleich weit beabstandet ist. Die zweite Elektrode 18 ist von einem einseitig geschlossenen Glasrohr 20 umgeben. Dabei ist das Glasrohr 20 an dem freien Stirnende der zweiten Elektrode 18 geschlossen und an seiner der Leiterplatte 14 zugewandten Seite 14 offen ausgebildet. Das Glasrohr 20 isoliert die Elektrode 18 gegenüber dem umgebenden Fluid und der ersten bzw. äußeren Elektrode 2.

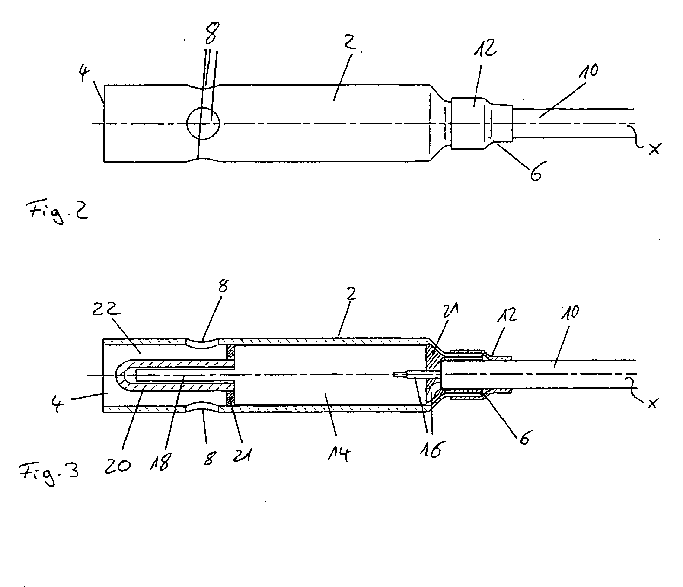

Fig. 2 zeigt eine Seitenansicht des zusammengesetzten Sensors. Die stirnseitige Öffnung 4, welche an der der elektrischen Anschlussleitung abgewandten Stirnseite in der äußeren Elektrode 2 ausgebildet ist sowie die umfänglichen Öffnungen 8 dienen zum Eintritt des Fluids in das Innere der äußeren Elektrode 2. Die umfänglichen Öffnungen 18 sind vorzugsweise gleichmäßig über den gesamten Umfang der Elektrode verteilt, um eine gute und gleichmäßige Durchströmung des Sensors von dem Fluid bzw. einen gleichmäßigen und stetigen Fluidaustausch im Inneren der äußeren Elektrode 2 zu gewährleisten. Der Isolationsschlauch 12 umgibt die Anschlussleitung 10 sowie das verjüngte Ende im Bereich der stirnseitigen Öffnung 6 der äußeren Elektrode 2, so dass die stirnseitige Öffnung 6 durch den Isolationsschlauch 12 nach außen abgedichtet ist. Zusätzlich dichtet der Isolationsschlauch 12 die elektrische Verbindung zwischen der Anschlussleitung 10 und der äußeren Elektrode 2 nach außen ab, so dass kein Fluid in das Innere der elektrischen Anschlussleitung 10 eintreten kann.

Im Bereich zwischen den umfänglichen Öffnungen 8 und der stirnseitigen Öffnung 6 können am Außenumfang der äußeren Elektrode 2 Befestigungs- und/oder Dichtungsmittel zum Einsetzen der äußeren Elektrode 2 in ein Gehäuse vorgesehen sein. So kann sich beispielsweise ein ringförmiger Flansch vom Außenumfang der äußeren Elektrode 2 radial nach außen erstrecken. Ferner kann am Außenumfang der äußeren Elektrode 2 ein Gewinde zum Befestigen des Sensors in einer Gehäusewandung ausgebildet sein.

Fig. 3 zeigt einen Längsschnitt durch den zusammengesetzten Sensor gemäß Fig. 2. Zu erkennen ist, dass sich die zweite Elektrode 18 entlang der Längsachse X des Sensors ausgehend von einer Stirnkante der Leiterplatte 14 zu der stirnseitigen Öffnung 4 der äußeren Elektrode 2 hin erstreckt. Die stabförmige zweite Elektrode 18 ist somit im Zentrum der rohrförmigen äußeren Elektrode 2 angeordnet. Dabei erstreckt sich die zweite Elektrode 18 insbesondere in den Bereich der äußeren Elektrode 2 zwischen den Umfangsöffnungen 8 und der stirnseitigen Öffnung 4.

Die Leiterplatte 14 ist im Inneren der äußeren Elektrode 2 in dem Bereich zwischen den umfänglichen Öffnungen 8 und der stirnseitigen Öffnung 6, durch welche sich die elektrische Anschlussleitung 10 erstreckt, angeordnet. Die Leiterplatte 14 weist eine Breite auf, welche im Wesentlichen dem Innendurchmesser der äußeren Elektrode 2 entspricht, so dass sie spielfrei in das Innere der äußeren Elektrode 2 eingesetzt werden kann. Auf diese Weise wird die fest mit der Leiterplatte 14 verbundene zweite Elektrode 18 in der vorgegebenen Position zentral im Inneren der äußeren Elektrode 2 fixiert. Der Bereich der äußeren Elektrode 2, in welchen die Leiterplatte 14 angeordnet ist, ist durch eine Vergussmasse 21, z. B. ein Harz, verfüllt. Die Vergussmasse 21 dichtet den Raum, in dem die Leiterplatte 14 eingesetzt ist nach außen und insbesondere zu dem Ringraum 22 zwischen dem Glasrohr 20 und dem Inneren der äußeren Elektrode 2 hin ab. Ferner sorgt sie für eine feste Fixierung der Leiterplatte 14 und damit der zweiten Elektrode 18 im Inneren der äußeren Elektrode 2. Das Glasrohr 20 greift mit seinem offenen Ende in die Vergussmasse 21 ein, so dass es dort dichtend mit der Vergussmasse 21 verbunden ist. Auf diese Weise ist die innere bzw. zweite Elektrode 18 gegen das in den Ringraum 2 eintretende Fluid isoliert. Die Vergussmasse 21 verschließt und isoliert ferner den Bereich im Inneren der äußeren Elektrode 2, in welchen die elektrische Anschlussleitung 10 eintritt. Auf diese Weise sind die Leiterplatte 14 und sämtliche elektrischen Anschlüsse und Verbindungen bzw. elektrischen Bauteile auf der Leiterplatte nach außen hin isoliert.

Das zu überwachende Fluid tritt durch die umfänglichen Öffnungen 8 sowie durch die stirnseitige Öffnung 4 in den Ringraum 22 ein und bildet somit das Dielektrikum in dem elektrischen Feld zwischen der äußeren Elektrode 2 sowie der inneren Elektrode 18. Die stirnseitige Öffnung 4 sowie die umfänglichen Öffnungen 8 ermöglichen einen guten Fluidaustausch; so dass auch die zu erfassenden Fremdstoffe, beispielsweise Wasser, schnell mit dem Fluid in das Innere der äußeren Elektrode 2, d. h. in den Messbereich des Sensors eintreten. Dabei erfolgt die Messung im direkten elektrischen Feld, welches sich radial zwischen der inneren Elektrode 18 und der äußeren Elektrode 2 erstreckt, was zu besonders guten Messergebnissen führt.

### Bezugszeichen

- 2: äußere Elektrode
- 4,6: stirnseitige Öffnungen
- 8: umfängliche Öffnung
- 10: elektrische Anschlussleitung
- 12: Isolationsschlauch
- 14: Leiterplatte
- 16: elektrische Leiter
- 18: innere Elektrode
- 20: Glasrohr
- 21: Vergussmasse
- 22: Ringraum

## Patentansprüche

1. Kapazitiver Sensor zum Erfassen eines Fremdstoffes in einem Fluid mit einer rohrförmigen ersten Elektrode (2) und einer zweiten Elektrode (18), welche im Inneren der ersten Elektrode (2) beabstandet zur Innenwandung der ersten Elektrode (2) angeordnet ist, **dadurch gekennzeichnet, dass** die zweite Elektrode (18) an einer Leiterplatte (14), welche im Inneren der ersten rohrförmigen Elektrode (2) angeordnet ist, zum elektrischen Anschluss derart angebracht ist, dass sich die zweite Elektrode (18) ausgehend von einer Stirnkante der Leiterplatte (14) in Längsrichtung der ersten Elektrode (2) zu einer stirnseitigen Öffnung (4) der ersten Elektrode (2) hin erstreckt.

2. Kapazitiver Sensor nach Anspruch 1, **dadurch gekennzeichnet, dass** die erste (2) und/oder die zweite Elektrode (18) von einer Isolierung (20) umhüllt ist.

3. Kapazitiver Sensor nach Anspruch 1 oder 2, **dadurch gekennzeichnet, dass** die zweite Elektrode (18) von einer Isolierung in Form eines, vorzugsweise an einem Ende geschlossenen, Glasrohres (20) umhüllt ist.

4. Kapazitiver Sensor nach einem der vorangehenden Ansprüche, **dadurch gekennzeichnet, dass** in der rohrförmigen ersten Elektrode (2) zumindest eine umfängliche Öffnung (8) vorgesehen ist.

5. Kapazitiver Sensor nach einem der vorangehenden Ansprüche, **dadurch gekennzeichnet, dass** ein Bereich im Inneren der ersten Elektrode (2), in welchem elektrische Anschlüsse und insbesondere eine Leiterplatte (14) angeordnet sind, mit einem Isolationsmaterial (21) ausgegossen ist und die zweite Elektrode (18) zumindest teilweise außerhalb des vergossenen Bereiches angeordnet ist.

6. Kapazitiver Sensor nach Anspruch 5, **dadurch gekennzeichnet, dass** in den vergossenen Bereich die die zweite Elektrode (18) umhüllende Isolierung (20) und/oder zumindest ein Zuleitungskabel (10) eingegossen ist.

7. Kapazitiver Sensor nach einem der vorangehenden Ansprüche, **dadurch gekennzeichnet, dass** ein stirnseitiger Endbereich der ersten Elektrode (2) eine Öffnung (6) aufweist, durch welche sich zumindest eine elektrische Anschlussleitung (10) in das Innere der ersten Elektrode (2) erstreckt.

8. Kapazitiver Sensor nach einem der vorangehenden Ansprüche, **dadurch gekennzeichnet, dass** die erste Elektrode (2) durch eine Klemmverbindung elektrisch leitend mit einer elektrischen Anschlussleitung (10) verbunden ist.

9. Kapazitiver Sensor nach einem der vorangehenden Ansprüche, **dadurch gekennzeichnet, dass** der Sensor zum Einbau mit der rohrförmigen Elektrode (2) in eine korrespondierende Öffnung eines Gehäuses dichtend einsetzbar ist.

10. Verwendung eines kapazitiven Sensors gemäß einem der vorangehenden Ansprüche in einer Flüssigkeitsvorlage eines in ein Fluid eintauchbaren Elektromotors.

## Claims

1. A capacitive sensor for detecting foreign matter in a fluid, with a tubular, first electrode (2) and with a second electrode (18) which is arranged in the inside of the first electrode (2) at a distance to the inner wall of the first electrode (2), **characterised in that** the second electrode (18), for electrical connection, is attached on a circuit board (14) which is arranged in the inside of the first tubular electrode (2), in manner such that the second electrode (18) departing from an end-edge of the circuit board (145) extends in the longitudinal direction of the first electrode (2) to an end-side opening (4) of the first electrode (2).

2. A capacitive sensor according to claim 1, **characterised in that** the first (2) and/or the second electrode (18) is enveloped by an insulation (20).

3. A capacitive sensor according to claim 1 or 2, **characterised in that** the second electrode (18) is enveloped by an insulation in the form of a glass tube (20), preferably closed at one end.

4. A capacitive sensor according to one of the preceding claims, **characterised in that** at least one peripheral opening (8) is provided in the tubular first electrode (2).

5. A capacitive sensor according to one of the preceding claims, **characterised in that** a region in the inside of the first electrode (2), in which region electrical connections and in particular a circuit board (14) are arranged, is cast out with an insulation material (21), and the second electrode (18) at least partly is arranged outside the cast region.

6. A capacitive sensor according to claim 5, **characterised in that** the insulation (20) enveloping the second electrode (18) and/or at least a supply cable (10) is cast into the cast region.

7. A capacitive sensor according to one of the preceding claims, **characterised in that** an end-side end region of the first electrode (2) comprises an opening (6) through which at least one electrical connection lead (10) extends into the inside of the first electrode (2).

8. A capacitive sensor according to one of the preceding claims, **characterised in that** the first electrode (2) by way of a terminal connection, is connected in an electrically conductive manner to an electrical connection lead (10).

9. A capacitive sensor according to one of the preceding claims, **characterised in that** the sensor for installation with the tubular electrode (2) may be sealingly inserted into a corresponding opening of a housing.

10. The use of a capacitive sensor according to one of the preceding claims in a fluid receptacle of an electric motor submersible into a fluid.

## Revendications

1. Capteur capacitif pour détecter une substance étrangère dans un fluide, comportant une première électrode (2) tubulaire et une deuxième électrode (18) disposée à l'intérieur de la première électrode (2) à distance de la paroi intérieure de la première électrode (2), **caractérisé en ce que** la deuxième électrode (18) est fixée sur un circuit imprimé (14) lui-même disposé à l'intérieur de la première électrode (2) tubulaire, pour son raccordement électrique, de telle sorte que la deuxième électrode (18) s'étend à partir d'une arête frontale du circuit imprimé (14) dans le sens longitudinal de la première électrode (2) vers une ouverture (4) frontale de la première électrode (2).

2. Capteur capacitif selon la revendication 1, **caractérisé en ce que** la première (2) et/ou la deuxième électrode (18) sont enveloppées d'une isolation (20).

3. Capteur capacitif selon la revendication 1 ou 2, **caractérisé en ce que** la deuxième électrode (18) est enveloppée d'une isolation sous forme d'un tube de verre (20), de préférence fermé à une extrémité.

4. Capteur capacitif selon l'une des revendications précédentes, **caractérisé en ce que**, dans la première électrode (2) tubulaire, il est prévu au moins une ouverture (8) périphérique.

5. Capteur capacitif selon l'une des revendications précédentes, **caractérisé en ce qu'**une zone à l'intérieur de la première électrode (2) dans laquelle sont disposés des connexions électriques, et en particulier un circuit imprimé (14), est remplie d'une matière isolante (21), et **en ce que** la deuxième électrode (18) est disposée au moins en partie à l'extérieur de la zone remplie.

6. Capteur capacitif selon la revendication 5, **caractérisé en ce que** l'isolation (20) enveloppant la deuxième électrode (18) et/ou au moins un câble d'alimentation (10) sont introduits dans la zone remplie.

7. Capteur capacitif selon l'une des revendications précédentes, **caractérisé en ce qu'**une zone terminale frontale de la première électrode (2) présente une ouverture (6) à travers laquelle au moins une ligne de branchement (10) électrique s'étend à l'intérieur de la première électrode (2).

8. Capteur capacitif selon l'une des revendications précédentes, **caractérisé en ce que** la première électrode (2) est reliée de façon électroconductrice à une ligne de branchement (10) électrique par un raccord à pince.

9. Capteur capacitif selon l'une des revendications précédentes, **caractérisé en ce que** le capteur peut être inséré de façon étanche dans une ouverture correspondante d'un boîtier pour le montage avec l'électrode (2) tubulaire.

10. Utilisation d'un capteur capacitif selon l'une des revendications précédentes, dans un dispositif hydraulique d'un moteur électrique immersible dans un fluide.
